# EUROPÄISCHE PATENTSCHRIFT

(11) **EP 4 495 089 B1**
(45) Veröffentlichungstag und Bekanntmachung des Hinweises auf die Patenterteilung: **04.03.2026**
(21) Anmeldenummer: 23186733.4
(22) Anmeldetag: 20.07.2023
(51) Int. Cl.: C07C 2/06, C07C 11/02, C07C 67/38, C07C 69/24, C07C 67/54

(54) **VERFAHREN ZUR ALKOXYCARBONYLIERUNG VON DI-ISOBUTEN UND EINEM C4- BIS C7-OLEFIN**
METHOD FOR ALKOXYCARBONYLATION OF DI-ISOBUTENE AND A C4-C7 OLEFIN
PROCÉDÉ D'ALCOXYCARBONYLATION DE DI-ISOBUTÈNE ET D'UNE OLÉFINE C4- À C7

(43) Veröffentlichungstag der Anmeldung: 22.01.2025
(73) Patentinhaber: Evonik Oxeno GmbH & Co. KG, 45772 Marl (DE)
(72) Erfinder: KUCMIERCZYK, Peter, 44628 Herne (DE); TERMÜHLEN, Maren, 44135 Dortmund (DE); REINHARDT, Annika, 45770 Marl (DE); RIX, Armin Matthias, 45770 Marl (DE); FRANKE, Robert, 45772 Marl (DE); FRIDAG, Dirk, 45721 Haltern an See (DE); SALE, Anna Chiara, 45657 Recklinghausen (DE); MARKOVIC, Ana, 45721 Haltern an See (DE)
(74) Vertreter: Evonik Patent Association

(56) Entgegenhaltungen:
- EP-A1- 3 750 620
- CA-A1- 2 973 451

## Beschreibung

Die vorliegende Erfindung betrifft ein Verfahren zur Alkoxycarbonylierung von Di-isobuten und einem C4- bis C7-Olefin in einer gemeinsamen Reaktionszone. Die Alkoxycarbonylierung wird mit einem Alkohol und Kohlenmonoxid in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, durchgeführt. Di-isobuten ist ein technisch relevantes Erzeugnis, welches durch Dimerisierung von Isobuten gewonnen wird. Di-isobuten besteht aus den Isomeren 2,4,4-Trimethylpent-1-en (nachfolgend auch: TMP1) und 2,4,4-Trimethylpent-2-en (nachfolgend auch: TMP2) mit einer Massenverteilung TMP1 : TMP2 im Bereich von etwa 78:22 bis 81 : 19 (Gleichgewichtsverteilung). Technische Gemische, die C4-Olefine enthalten, sind Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. C5-Olefine, also Pentene, sind in Leichtbenzinfraktionen aus Raffinerien oder Crackern enthalten. Die höheren Olefine können insbesondere durch Oligomerisierungsreaktionen gewonnen werden. Sowohl Di-isobuten als auch C4- bis C7-Olefine können durch Alkoxycarbonylierung zu Wertprodukten wie den bei der Alkoxycarbonylierung gebildeten Estern umgesetzt werden. Das Dokument EP 3 750 620 A1 offenbart ein Verfahren zur Alkoxycarbonylierung von Diisobutylen. Das Dokument CA 2 973 451 A1 offenbart ein Verfahren zur Alkoxycarbonylierung eines Olefingemisches.

Das Problem bei derartigen Verfahren ist, dass eigenständige Produktionsanlagen vorhanden oder gebaut werden und dass diese Anlagen mit entsprechendem Aufwand betrieben werden müssen.

Da die Märkte für petrochemische Produkte zum Teil recht volatil sein, sind eigene Produktionsanlagen für jede der genannten Olefine kaum wirtschaftlich zu betrieben. Ein weiterer Nachteil ist, dass ein ressourcenschonender Betrieb von mehreren Produktionsanlagen kaum möglich ist, weil alle Anlagen instandgehalten werden müssen. Das ist nicht nur ein wirtschaftlicher und personeller Aufwand, sondern erfordert auch gewisse Mengen an Energie wie Strom oder Wärmeträger.

Die Aufgabe der vorliegenden Erfindung war deshalb die Bereitstellung eines Verfahrens, welches die vorgenannten Probleme nicht aufweist. Insbesondere sollten sowohl Di-isobuten als auch C4-bis C7-Olefine per Alkoxycarbonylierung ressourcenschonender zu Wertprodukten umgesetzt werden können.

Die zugrundeliegende Aufgabe konnte durch das in Anspruch 1 beschriebene Verfahren gelöst werden. Bevorzugte Ausführungsformen sind in den abhängigen Ansprüchen angegeben.

Erfindungsgemäß umfasst das Verfahren zur Alkoxycarbonylierung von Di-isobuten und einem C4-bis C7-Olefin zumindest die folgenden Schritte:
a. Bereitstellen eines Di-isobutenstroms, enthaltend 2,4,4-Trimethylpent-2-en und 2,4,4-Trimethylpent-1-en, und Bereitstellen eines Olefinstroms, enthaltend das C4- bis C7-Olefin, vorzugsweise C4-Olefine;
b. Alkoxycarbonylierung des Di-isobutens und des C4- bis C7-Olefins, vorzugsweise C4-Olefins mit einem Alkohol und Kohlenmonoxid in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, in einer gemeinsamen Reaktionszone unter Erhalt eines flüssigen Produktgemisches, welches zumindest die durch die Alkoxycarbonylierung gebildeten Ester, das homogene Katalysatorsystem, nicht umgesetzte Olefine, d. h. nicht umgesetztes Di-isobuten und nicht umgesetzte C4- bis C7-Olefine, und nicht umgesetzten Alkohol umfasst;
c. Abtrennen des homogenen Katalysatorsystems aus dem flüssigen Produktgemisch unter Erhalt eines Rohproduktgemisches, welches zumindest die durch die Alkoxycarbonylierung gebildeten Ester, die nicht umgesetzten Olefine und nicht umgesetzte Alkohole umfasst; und
d. Destillative Aufarbeitung des Rohproduktgemisches in mindestens einer Destillationskolonne zur Abtrennung der nicht umgesetzten Alkohole und der nicht umgesetzten Olefine unter Erhalt eines Estergemisches, welches die gebildeten Ester enthält, wobei vorzugsweise nicht umgesetzter Alkohol und nicht umgesetzte Olefine abgetrennt und zur Alkoxycarbonylierung in Schritt b zurückgeführt wird.

Das erfindungsgemäße Verfahren betrifft also die gleichzeitige Umsetzung von Di-isobuten und C4- bis C7-Olefinen in einer einzigen gemeinsamen Reaktionszone. Ein solches Verfahren hat eine Vielzahl von Vorteilen.

Durch das beschriebene Verfahren ist es möglich, dass auf Märkte, insbesondere geringer Produktionsmengen, flexibel reagiert werden kann. Zudem ist nur eine Produktionsanlage notwendig, die zudem selbst bei schwankenden Marktbedürfnissen effizienter und damit ressourcenschonender betrieben werden kann. Durch die Besonderheit der Siedereihenfolge ist es außerdem möglich, die anfallenden Produkte der jeweils eingesetzten Olefine, sowie dem Lösungsmittel Methanol zu trennen, während die Edukte wieder direkt oder nach zusätzlicher Aufarbeitung der Reaktion zugeführt werden können.

Der in Schritt a bereitgestellte Di-isobutenstrom enthält 2,4,4-Trimethylpent-2-en und 2,4,4-Trimethylpent-1-en. In einer bevorzugten Ausführungsform beträgt der Anteil von 2,4,4-Trimethylpent-1-en im Di-isobutenstrom mindestens 60 Mol%, vorzugsweise mindestes 70 Mol% bezogen auf den gesamten Di-isobutenstrom. Derartige Ströme können Di-isobutenströme sein, die mittels Dimerisierung aus Isobuten oder Isobuten-haltigen Kohlenwasserstoffgemischen hergestellt wurden, beispielsweise nach dem in EP 1 360 160 B1 offenbarten Verfahren. Weiterhin können die hier einzusetzenden Di-isobutenströme als nicht reagierte Restströme bei Carbonylierungsverfahren anfallen, beispielsweise bei der Alkoxycarbonylierung oder bei der Hydroformylierung.

Neben dem Di-isobutenstrom wird in Schritt a ein Olefinstrom bereitgestellt, der das im erfindungsgemäßen Verfahren eingesetzte C4- bis C7-Olefin enthält. In einer bevorzugten Ausführungsform wird in der vorliegenden Erfindung ein Olefinstrom eingesetzt, der C4-Olefine enthält, besonders bevorzugt ein C4-Olefinstrom. Entsprechende Ströme sind dem Fachmann bekannt und großtechnisch verfügbar

Olefineströme, die C4-Olefine enthalten, sind beispielsweise Leichtbenzinfraktionen aus Raffinerien, C4-Fraktionen aus FC- oder Steamcrackern, Gemische aus Fischer-Tropsch-Synthesen, Gemische aus Dehydrierung von Butanen oder durch Metathese oder aus anderen technischen Prozessen entstandene Ströme. Beispielsweise können für das erfindungsgemäße Verfahren geeignete C4-Olefinströme aus der C4-Fraktion eines Steamcrackers gewonnen werden. C5-Olefine, also Pentene, sind in Leichtbenzinfraktionen aus Raffinerien oder Crackern enthalten.C6-Olefine können beispielsweise durch Dimerisierung von Propen gewonnen werden. C7-Olefine können beispielsweise durch Dimerisierung von Propylen und Buten gewonnen werden.

Die in Schritt a bereitgestellten Ströme, d. h. der Di-isobutenstrom und der C4- bis C7-Olefinstrom werden zur Alkoxycarbonylierung in Schritt b geleitet. Dabei können die Ströme einzeln und separat zur Alkoxycarbonylierung in Schritt b geleitet werden oder vorher vermischt werden. Vorzugsweise werden der Di-isobutenstrom und der C4- bis C7-Olefinstrom vor der Alkoxycarbonylierung in Schritt b vermischt. In einer besonders bevorzugten Ausführungsform der vorliegenden Erfindung werden sogar der Di-isobutenstrom, der C4- bis C7-Olefinstrom, der Alkohol und das homogene Katalysatorsystem vor der Alkoxycarbonylierung in Schritt b vermischt, insbesondere in einem geeigneten Mischbehälter. Sofern es einen Recyclestrom zur Reaktion gibt, also z. B. durch Rückführung des Katalysatorsystems, kann dieser Recyclestrom ebenfalls zum Mischbehälter geführt werden.

Die Di-isobutene, d. h. 2,4,4-Trimethylpent-2-en und 2,4,4-Trimethylpent-1-en, und das C4- bis C7-Olefin werden in Schritt b mit Kohlenmonoxid (CO) und einem Alkohol zu einem Ester umgesetzt.

Die Anzahl der Kohlenstoffatome im Ester erhöht sich dabei im Vergleich mit dem eingesetzten Olefin um 1 Kohlenstoffatom aus dem Kohlenmonoxid sowie um die Anzahl der Kohlenstoffatome des eingesetzten Alkohols. Aus den Di-isobutenen (8 Kohlenstoffatome) entsteht demnach ein Ester mit 9 Kohlenstoffatomen im Säureteil des Esters plus den Kohlenstoffatomen des Alkohols im Alkoholteil des Esters.

Das Kohlenmonoxid kann bei der Alkoxycarbonylierung in Schritt b direkt als Einsatzgemisch oder durch die Zugabe eines Kohlenmonoxid-haltigen Gases, ausgewählt aus Synthesegas, Wassergas, Generatorgas und anderen Kohlenmonoxid-haltigen Gasen bereitgestellt werden. Möglich ist es auch, das Kohlenmonoxid dadurch bereitzustellen, dass das Kohlenmonoxid-haltige Gas vorher in einer für den Fachmann bekannten Weise in seine Komponenten aufgetrennt wird und das Kohlenmonoxid zur Reaktionszone geleitet wird. Das Kohlenmonoxid kann dabei weiterhin einen gewissen Anteil an Wasserstoff oder anderen Gasen enthalten, weil eine vollständige Auftrennung kaum zu realisieren ist.

Der bei der Umsetzung in Schritt b eingesetzte Alkohol ist vorzugsweise ein einwertiger Alkohol mit 1 bis 4 Kohlenstoffatomen, insbesondere Methanol, Ethanol, Propanol oder Butanol. Dadurch werden dann die Ester mit dem eingesetzten Alkohol entsprechender Kettenlänge erhalten. Bei Einsatz von Methanol entsteht beispielsweise der Methylester.

Der in Schritt b eingesetzte Alkohol, vorzugsweise der einwertige Alkohol mit 1 bis 4 Kohlenstoffatomen, insbesondere Methanol, Ethanol, Propanol oder Butanol, wird in einem Molverhältnis zur Gesamtmenge aller Olefine, d. h. der Di-isobutene und der C4- bis C7-Olefine, Alkohol : Olefin in einem Bereich von 10 : 1 bis 1 : 1, vorzugsweise 8 : 1 bis 1,5 : 1, besonders bevorzugt 7 : 1 bis 2 : 1 eingesetzt. Der Alkohol wird somit - bezogen auf die eingesetzten Olefine - mindestens in identischer molarer Menge, vorzugsweise aber im molaren Überschuss hinzugefügt.

Die erfindungsgemäße Umsetzung in Schritt b wird in Gegenwart eines homogenen Katalysatorsystems durchgeführt, welches zumindest ein Metall aus der Gruppe 8 bis 10 des Periodensystems der Elemente (PSE) oder eine Verbindung davon, einen phosphorhaltigen Liganden und eine Säure als Co-Katalysator umfasst. Es versteht sich, dass ein geeigneter Katalysator in der Lage sein muss, alle Olefine, d. h. die Di-isobutene und die C4- bis C7-Olefine, zu alkoxycarbonylieren. Der Gehalt des Metalls der Gruppe 8 bis 10 des Periodensystems der Elemente, insbesondere des Palladiums, beträgt in der der Reaktionslösung bei der Alkoxycarbonylierung in Schritt b vorzugsweise 100 bis 500 ppm, weiterhin bevorzugt 150 bis 450 ppm, besonders bevorzugt 180 bis 350 ppm.

Das Metall aus der Gruppe 8 bis 10 des PSE ist vorzugsweise Palladium. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladiumchlorid [PdCl₂], Palladium(II)-Acetylacetonat [Pd(acac)₂], Palladium(II)-Acetat [Pd(OAc)₂], Dichloro-(1,5-cyclooctadien)palladium(II) [Pd(cod)₂Cl2], Bis(dibenzylideneaceton)palladium(0) [Pd(dba)₂], Tris(dibenzylideneaceton)dipalladium(0) [Pd₂(dba)₃] Bis(acetonitril)-dichloropalladium(II) [Pd(CH₃CN)₂Cl₂], Palladium(cinnamyl)-dichlorid [Pd(cinnamyl)Cl₂]. Vorzugsweise kommen die Verbindungen [Pd(acac)₂] oder [Pd(OAc)₂] zum Einsatz. Die Metallkonzentration von Palladium in Schritt b beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für die das erfindungsgemäße Katalysatorsystem sind benzolbasierte Diphosphinverbindungen, wie sie beispielsweise in der EP 3 121 184 A2 offenbart worden sind. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall kann für die beschriebene Umsetzung in Schritt b 1 : 1 bis 10 : 1, vorzugsweise 2 : 1 bis 6 : 1, besonders bevorzugt 3 : 1 bis 5 : 1 betragen.

Das homogene Katalysatorsystem umfasst weiterhin eine Säure, wobei es sich insbesondere um eine Brönsted- oder eine Lewis-Säure handelt. Als Lewis-Säure werden vorzugsweise Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von pKs ≤ 5, besonders bevorzugt eine Säurestärke von pKs ≤ 3. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 5 : 1 hinzugegeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure.

Die Alkoxycarbonylierung in Schritt b wird vorzugsweise bei einer Temperatur im Bereich von 60 bis 120 °C, weiterhin bevorzugt im Bereich von 65 bis 110 °C, besonders bevorzugt im Bereich von 70 bis 100 °C durchgeführt. Die Alkoxycarbonylierung in Schritt b wird weiterhin bevorzugt bei einem Kohlenmonoxiddruck von 10 bis 35 bar, vorzugsweise 12,5 bis 30 bar, besonders bevorzugt 15 bis 25 bar durchgeführt.

Die Alkoxycarbonylierung in Schritt b findet in einer geeigneten Reaktionszone statt. Die Reaktionszone für die Umsetzung umfasst mindestens einen Reaktor, kann aber auch aus zwei oder mehr Reaktoren bestehen, die parallel oder in Reihe geschaltet angeordnet sind. Der mindestens eine Reaktor kann insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein.

Durch die oben beschriebene Alkoxycarbonylierung in Schritt b wird ein flüssiges Produktgemisch erhalten, das zumindest die durch die Alkoxycarbonylierung gebildeten Ester, das homogene Katalysatorsystem, nicht umgesetzte Olefine und nicht umgesetzten Alkohol umfasst. Zusätzlich kann das flüssige Produktgemisch leichtsiedende Nebenprodukte wie Dimethylether oder Ameisensäure und/oder hochsiedende Komponenten wie Ligandenabbauprodukte enthalten.

Das so erhaltene Produktgemisch wird dem nachfolgenden Schritt c zugeführt, um das homogene Katalysatorsystems aus dem flüssigen Produktgemisch abzutrennen. Vor dem Zuführen des Produktgemisch können bereits leichtsiedende Komponenten beispielsweise ein Teil des nicht umgesetzten Alkohols und/oder der leichtsiedenden Nebenprodukte abgetrennt werden, beispielsweise mittels Destillation. Der Alkohol kann zu Schritt b, also der Alkoxycarbonylierung zurückgeführt werden. Die leichtsiedenden Nebenprodukte können aus dem Verfahren im Sinne eines Purge ausgeschleust werden, damit sie sich nicht im Verfahren anreichern. Aus der Abtrennung des homogenen Katalysatorsystems wird ein Rohproduktgemisch erhalten, welches zumindest die durch die Alkoxycarbonylierung gebildeten Ester, nicht umgesetzte Olefine und zumindest einen Teil der nicht umgesetzten Alkohole umfasst.

Die Abtrennung des homogenen Katalysatorsystems unter Erhalt des Rohproduktgemisches in Schritt c kann mithilfe verschiedener Trennverfahren erfolgen, beispielsweise mittels thermischer Trennung oder durch Membrantrennung. Entsprechende Verfahren sind dem Fachmann geläufig. Vorzugsweise erfolgt die Abtrennung des homogenen Katalysatorsystems in Schritt c im Rahmen der vorliegenden Erfindung vorzugsweise mittels Membrantrennung. Bei einer Membrantrennung fallen bekanntermaßen ein Retentat und ein Permeat an. Im Retentat wird sich das homogene Katalysatorsystem anreichern. Das Permeat stellt dann das bereits erwähnte Rohproduktgemisch dar und wird zum nachfolgenden Schritt d, der destillativen Aufarbeitung geleitet.

Es ist erfindungsgemäße bevorzugt, dass das Retentat zur Alkoxycarbonylierung in Schritt b bzw. zur Reaktionszone, wo die Alkoxycarbonylierung durchgeführt wird, zurückgeführt wird. So kann das Katalysatorsystem wiederverwendet werden. Bei der bevorzugt kontinuierlichen Durchführung des beanspruchten Verfahrens entsteht so ein Katalysatorkreislauf, wo wenn überhaupt nur geringfügige prozessbedinge Katalysatorverluste ausgeglichen werden müssen. Sofern der Di-isobutenstrom, der Olefinstrom, der Alkohol und das homogene Katalysatorsystem gemäß der bevorzugten Ausführungsform vor der Alkoxycarbonylierung in Schritt b insbesondere in einem geeigneten Mischbehälter vermischt werden wird, wird das Retentat zum Mischbehälter geführt. Bei der Rückführung des Retentats kann weiterhin ein Purgestrom, welcher inerte Alkane, leichtsiedende Nebenprodukte (bspw. Ether), mögliche Abbauprodukte des Katalysatorsystems oder andere Verunreinigungen wie beispielsweise Spuren von Wasser oder Stickstoff enthalten kann, entnommen werden, um eine Akkumulation in der oder den Reaktionszonen zu vermeiden.

Bei der Membrantrennung kann jedes geeignete Membranmaterial eingesetzt werden. Vorzugsweise wird bei der Membrantrennung in Schritt c des erfindungsgemäßen Verfahrens ein OSN-Membranmaterial (OSN = Organic Solvent Nanofiltration) eingesetzt. Ein solches Membranmaterial besteht vorzugsweise zumindest aus einer trennaktiven Schicht (auch: aktiven Trennschicht) und einer Unterstruktur, worauf sich die trennaktive Schicht befindet. Bevorzugt besteht das erfindungsgemäße Membranmaterial zumindest aus einer trennaktiven Schicht und einer Unterstruktur.

Das Membranmaterial, aus zumindest trennaktiver Schicht und einer Unterstruktur, sollte säurestabil sein, damit das Membranmaterial nicht durch die im flüssigen Produktgemisch befindliche Säure beschädigt wird. Der Begriff "säurestabil" meint im Rahmen der vorliegenden Erfindung, dass das Membranmaterial mindestens 300 h in Anwesenheit der Säure des Katalysatorsystems stabil ist und die Trennleistung erhalten bleibt.

Die Unterstruktur weist vorzugsweise eine poröse Struktur auf, die für das durch die trennaktive Schicht gelangte Permeat durchlässig ist. Die Unterstruktur hat eine stabilisierende Funktion und dient als Träger für die trennaktiven Schicht. Die Unterstruktur kann grundsätzlich aus jedem geeigneten porösen Material bestehen. Entsprechende Materialien sind dem Fachmann geläufig. Voraussetzung ist jedoch, dass das Material säure- und basenstabil ist. Die Unterstruktur kann auch aus dem gleichen Material bestehen wie die trennaktive Schicht. Bevorzugte Materialien für die Unterstruktur sind Kunststoffe wie Polypropylen (PP), Polyethylen (PE) oder nicht-Kondensationspolymere, die sich nicht hydrolytisch oder alkoholytisch spalten lassen, wie Polysulfone, Polytetrafluorethylen (PTFE), Polyethersulfon (PES), Polyvinylidenfluorid (PVDF) oder Polyacrylnitril (PAN).

Die erfindungsgemäße trennaktive Schicht besteht vorzugsweise aus einem PAEK-Polymer (Polyaryletherketon). PAEK zeichnet sich dadurch aus, dass innerhalb der Widerholungseinheit Arylgruppen abwechselnd über eine Etherfunktionalität und eine Ketonfunktionalität verknüpft sind. Eine erfindungsgemäß bevorzugte trennaktive Schicht besteht aus PEEK (Polyetheretherketon). Als trennaktive Schicht können insbesondere bevorzugt PEEK-Polymere mit einem Sulfonierungsgrad von weniger als 20%, besonders bevorzugt mit einem Sulfonierungsgrad von weniger als 10% eingesetzt werden. Die entsprechenden PEEK-Polymere und deren Herstellung sind in der WO 2015/110843 A1 beschrieben.

Die Membrantrennung in Schritt c wird vorzugsweise bei einer Temperatur im Bereich von 25 bis 100°C, weiterhin bevorzugt im Bereich von 30 bis 80°C und besonders bevorzugt im Bereich von 40 bis 70°C durchgeführt. Um das Produktgemisch auf die bei der Membrantrennung bevorzugte vorherrschende Temperatur zu bringen, kann das Produktgemisch gekühlt werden. Neben einer aktiven Kühlung unter Verwendung eines Kühlmediums, kann die Kühlung auch über einen Wärmetauscher erreicht werden, wo Wärmeenergie auf einen anderen Strom übertragen wird, wodurch sich das Produktgemisch abkühlt und der andere Strom erhitzt wird.

Der Transmembrandruck (TMP) liegt bei der Membrantrennung in Schritt c vorzugsweise im Bereich von 10 bis 60 bar, weiterhin bevorzugt im Bereich von 15 bis 55 bar, besonders bevorzugt im Bereich von 20 bis 50 bar. Der permeatseitige Druck kann dabei oberhalb des atmosphärischen Drucks liegen und vorzugsweise bis 15 bar, vorzugsweise 3 bis 7 bar betragen. Aus der Differenz von TMP und permeatseitigem Druck ergibt sich der retentatseitige Druck. In einer bevorzugten Ausführungsform sollte bei den Druckverhältnissen und insbesondere beim permeatseitigen Druck darauf geachtet werden, dass der Druck in Abhängigkeit von dem eingesetzten Kohlenwasserstoff, dem eingesetzten Alkohol und der vorhandenen Temperatur so eingestellt wird, dass eine Verdampfung nach dem Durchtritt durch die Membran vermieden wird. Ein Verdampfen könnte zu einer instabilen Fahrweise führen.

Im anschließenden Schritt d erfolgt die destillative Aufarbeitung des Rohproduktgemisches bzw. des Permeats aus der Membrantrennung in mindestens einer Destillationskolonne zur Abtrennung der nicht umgesetzten Alkohole und der nicht umgesetzten Olefine, d. h. nicht umgesetzte Di-isobutene und nicht umgesetzte C4- bis C7-Olefine. Dabei wird ein Estergemisch erhalten, das die gebildeten Ester enthält. Das Estergemisch enthält also die aus dem Di-isobuten und dem C4- bis C7-Olefin gebildeten Ester.

Bei der destillativen Aufarbeitung des Rohproduktgemisches bzw. des Permeats in Schritt d fallen der nicht umgesetzte Alkohol und die nicht umgesetzten Olefine, d. h. nicht umgesetzte Di-isobutene und nicht umgesetzte C4- bis C7-Olefine, am Kopf der mindestens einen Destillationskolonne an. Das Gemisch der gebildeten Ester fällt folglich im Sumpf der mindestens einen Destillationskolonne an. Der Kopfstrom, der den in der mindestens einen Destillationskolonne abgetrennten nicht umgesetzten Alkohol und die nicht umgesetzten Olefine enthält, können zur Alkoxycarbonylierung in Schritt b bzw. zur Reaktionszone zurückgeführt werden. Ist eine Vermischung der eingesetzten Komponenten vor der Alkoxycarbonylierung vorhanden, wird der Kopfstrom natürlich zur Vermischung geführt. Dadurch ist ein kontinuierlicher Betrieb des erfindungsgemäßen Verfahrens unter höchstmöglicher Ausbeute möglich. Aus dem zurückgeführten Kopfstrom kann ein Purge abgezogen werden, um leichtsiedende Nebenprodukte aus dem Verfahren auszuschleusen.

Die destillative Aufarbeitung zur Abtrennung der nicht umgesetzten Alkohole und der nicht umgesetzten Olefine in Schritt d kann in einer Destillationskolonne erfolgen. Denkbar wäre, dass die destillative Aufarbeitung zur Abtrennung der nicht umgesetzten Alkohole und der nicht umgesetzten Olefine in Schritt d in mehreren Destillationskolonnen erfolgt. Dies würde aber einen deutlichen höheren apparativen Aufwand bedeuten. Bevorzugt ist deshalb, dass die destillative Aufarbeitung in Schritt d in einer einzigen Destillationskolonne erfolgt.

Der Druck in der Destillationskolonne bei der destillativen Aufarbeitung in Schritt d liegt vorzugsweise im Bereich von 0,3 bis 2 bar, weiterhin bevorzugt im Bereich von 0,4 bis 1 bar, besonders bevorzugt im Bereich 0,5 bis 0,7 bar beträgt. Die Temperatur im Sumpf der Destillationskolonne bei der destillativen Aufarbeitung in Schritt d liegt vorzugsweise im Bereich von 80 °C bis 160 °C. Die Temperatur am Kopf der Destillationskolonne bei der destillativen Aufarbeitung in Schritt d liegt vorzugsweise im Bereich von 30 bis 80 °C. Weiterhin ist es bevorzugt, dass das Rücklaufverhältnis in der Destillationskolonne zwischen 1 und 2 beträgt. Die Destillationskolonne für die Abtrennung in Schritt d umfasst vorzugsweise 10 bis 30 theoretische Stufen umfasst. Die Destillationskolonne kann dabei Hochleistungsstrukturpackungen enthalten. Entsprechende Hochleistungsstrukturpackungen sind dem Fachmann bekannt.

Durch die destillative Aufarbeitung wird, wie erwähnt, ein Estergemisch erhalten, die die aus dem Di-isobuten und dem C4- bis C7-Olefin gebildeten Ester enthält. Um beide Ester als möglichst reine Stoffe zu erhalten kann ein weiterer Destillationsschritt durchgeführt werden, um die Ester aus dem Estergemisch voneinander zu trennen. Die Ester aus dem C4- bis C7-Olefin wird dabei am Kopf der Destillationskolonne und die Ester aus dem Di-isobuten am Sumpf der Destillationskolonne anfallen.

Das vorliegende Verfahren eignet sich zur Alkoxycarbonylierung von Di-isobuten und C4-Olefinen mit einem Alkohol, vorzugsweise einem Alkohol mit 1 bis 4 Kohlenstoffatomen. Bestimmte Kombinationen von Olefinen und Alkoholen sind im Rahmen der vorliegenden Erfindung besonders bevorzugt:
In einer bevorzugten Ausführungsform betrifft das Verfahren die Alkoxycarbonylierung von Di-isobuten und einem C4-Olefin, d. h. 1-Buten, cis- und/oder trans-2-Buten, Isobuten oder Mischungen davon, mit Methanol. Der aus Di-isobuten bei der Alkoxycarbonylierung mit Methanol gebildete Ester ist 3,5,5-Trimethylhexansäuremethylester. Aus den verschiedenen Butenen können durch die Alkoxycarbonylierung mit Methanol Valeriansäuremethylester, 2-Methylbuttersäuremethylester und/oder 3-Methylbuttersäuremethylester entstehen. Wird ein Gemisch von Butenen eingesetzt wird dementsprechend auch eine Mischung der genannten Ester erhalten.

In einer bevorzugten Ausführungsform betrifft das Verfahren die Alkoxycarbonylierung von Di-isobuten und einem C4-Olefin, d. h. 1-Buten, cis- und/oder trans-2-Buten, Isobuten oder Mischungen davon, mit Ethanol. Der aus Di-isobuten bei der Alkoxycarbonylierung mit Ethanol gebildete Ester ist 3,5,5-Trimethylhexansäureethylester. Aus den verschiedenen Butenen können durch die Alkoxycarbonylierung mit Ethanol Valeriansäureethylester, 2-Methylbuttersäureethylester und/oder 3-Methylbuttersäureethylester entstehen. Wird ein Gemisch von Butenen eingesetzt wird dementsprechend auch eine Mischung der genannten Ester erhalten.

Die vorliegende Erfindung wird nachfolgend anhand von Beispielen erläutert. Die Beispiele betreffen bevorzugte Ausführungsformen, sind aber nicht als die Erfindung einschränkend zu verstehen.

### Beispiele

### Beispiel 1 - Umsetzung von Di-isobuten und 1-Buten

Im vorliegenden Versuch werden als Substrate Di-isobuten, hier ein Gemisch aus,4,4-Trimethylpent-1-en (TMP1) und 2,4,4-Trimethylpent-2-en (TMP2) im molaren Verhältnis von 79:21 (TMP1:TMP2) und 1-Buten eingesetzt. Als Alkohol wird Methanol eingesetzt. Das bei der Alkoxycarbonylierung eingesetzte Katalysatorsystem umfasst die folgenden Substanzen: Als Säure kommt Aluminium-trifluoromethansulfonsäure (Al(OTf)₃) zum Einsatz. Als Ligand wurde 1,2-bis((tert-butyl(pyridin-2-yl)phosphanyl)methyl)benzen (L1) und als Präkursor für das Metall wird Palladium(II)bis(acetylacetonate) (Pd(acac)₂) eingesetzt. Zur späteren Quantifizierung wird Ethylbenzol als interner Standard verwendet.

Zunächst wird eine Katalysatorstammlösungen mit Methanol als Lösungsmittel mit einem Zielgehalt an Palladium von ca. 250 ppm angesetzt. Das Liganden:Pd-Verhältnis (molar) beträgt 4:1 sowie das Al(OTf)₃-Liganden-Verhältnis (molar) von 5:1. Diese Lösung wird 30 min bei Raumtemperatur unter Argon-Atmosphäre gerührt. Anschließend wird ein Teil dieser Lösung in einen Reaktor überführt. Zusätzlich wird Di-iso-Buten zugegeben. Nach dem Verschließen des Reaktors wird abwechselnd (3 mal) mit Argon auf bis zu 10 bar aufgedrückt und wieder entspannt, um Sauerstoff zu verdrängen. Dann wird ein Druck von 3 bar an Argon angelegt und die Reaktionslösung unter Rühren auf eine Temperatur von 80°C aufgeheizt. Ein zuvor an den Reaktor angeschlossenes Gefäß, das mit 1-Buten gefüllt ist, ist über Kopf mit einem Ventil verbunden, dass eine Begasung mit CO erlaubt. Über den Boden lässt sich bei Betätigen des Ventils der Inhalt des Gefäßes und CO in das Reaktionsgefäß überführen.

Zunächst wird ein Druck von 30 bar an CO im Gefäß eingestellt. Zum Reaktionsstart wird das das Ventil geöffnet, wodurch 1-Buten und CO in das Reaktionsgefäß gelangen.Das CO wird kontinuierlich zudiosiert, um einen konstanten Druck von 20 bar während der Reaktion zu erreichen. Der Reaktionsverlauf wird durch Probennahme überwacht. Die Proben werden mit Isopropanol verdünnt und mittels Gaschromatographie vermessen. Eine vorherige Kalibrierung der relevanten Komponenten erlaubt die Quantifizierung mittels enthaltenem Standard Ethylbenzol. Die Ergebnisse sind in Tabelle 1 zu sehen:

**Tabelle 1: Reaktionsverlauf nach Beispiel 1**

| Zeitpunkt [min] | Ausbeute 3,5,5-Trimethylhexansäuremethylester (TMH Methylester) [%] | Ausbeute n-Valeriansäuremethylester [%] | Ausbeute 2-Methylbuttersäuremethylester [%] | Ausbeute C5-Methylester* [%] | n/iso C5-Ester* [%] |
|---|---|---|---|---|---|
| 6 | 8 | 49 | 11 | 60 | 81:19 |
| 10 | 10 | 61 | 13 | 74 | 82:18 |
| 30 | 26 | 80 | 18 | 98 | 82:18 |
| 60 | 44 | 81 | 18 | 99 | 82:18 |
| 180 | 70 | 81 | 19 | 100 | 81:19 |
| 720 | 93 | 81 | 19 | 100 | 81:19 |

| | | | | | |
|---|---|---|---|---|---|
| * Summe der möglichen C5-Ester bezogen auf den Einsatzstoff | | | | | |

### Beispiel 2 - Umsetzung von Di-isobuten und trans-2-Buten

Der Versuch in Beispiel 2 wurde anhand der in Beispiel 1 beschriebenen Versuchsvorschrift durchgeführt. Der einzige Unterschied war der Einsatz von trans-2-Buten statt 1-Buten. Die Ergebnisse sind in Tabelle 2 gezeigt:

**Tabelle 2: Reaktionsverlauf nach Beispiel 2**

| Zeitpunkt [min] | Ausbeute 3,5,5-Trimethylhexansäuremethylester (TMH Methylester) [%] | Ausbeute n-Valeriansäuremethylester [%] | Ausbeute 2-Methylbuttersäuremethylester [%] | Ausbeute C5-Methylester* [%] | n/iso C5-Ester* [%] |
|---|---|---|---|---|---|
| 6 | 9 | 35 | 8 | 43 | 81:19 |
| 10 | 12 | 49 | 12 | 61 | 80:20 |
| 30 | 30 | 78 | 19 | 97 | 80:20 |
| 60 | 46 | 80 | 20 | 100 | 80:20 |
| 180 | 70 | 80 | 20 | 100 | 80:20 |
| 720 | 92 | 80 | 20 | 100 | 80:20 |

| | | | | | |
|---|---|---|---|---|---|
| * Summe der möglichen C5-Ester bezogen auf den Einsatzstoff | | | | | |

### Beispiel 3 - Umsetzung von Di-isobuten und Isobuten

Der Versuch in Beispiel 3 wurde anhand der in Beispiel 1 beschriebenen Versuchsvorschrift durchgeführt. Der einzige Unterschied war der Einsatz von Isobuten statt 1-Buten. Die Ergebnisse sind in Tabelle 3 gezeigt:

**Tabelle 3: Reaktionsverlauf nach Beispiel 3**

| Zeitpunkt [min] | Ausbeute 3,5,5-Trimethylhexansäuremethylester (TMH Methylester) [%] | Ausbeute 3-Methylbuttersäuremethylester [%] |
|---|---|---|
| 6 | 13 | 46 |
| 10 | 17 | 62 |
| 30 | 32 | 92 |
| 60 | 50 | 100 |
| 180 | 71 | 100 |
| 720 | 92 | 100 |

Die durchgeführten Versuche belegen, dass die gestellte Aufgabe durch eine erfindungsgemäße Verbindung gelöst wird.

## Patentansprüche

1. Verfahren zur Alkoxycarbonylierung von Di-isobuten und einem C4- bis C7-Olefin, wobei das Verfahren zumindest die folgenden Schritte umfasst:
a. Bereitstellen eines Di-isobutenstroms, enthaltend 2,4,4-Trimethylpent-2-en und 2,4,4-Trimethylpent-1-en, und Bereitstellen eines Olefinstroms, enthaltend das C4- bis C7-Olefin;
b. Alkoxycarbonylierung von Di-isobuten und dem C4- bis C7-Olefin mit einem Alkohol und Kohlenmonoxid in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, in einer gemeinsamen Reaktionszone unter Erhalt eines flüssigen Produktgemisches, welches zumindest die durch die Alkoxycarbonylierung gebildeten Ester, das homogene Katalysatorsystem, nicht umgesetzte Olefine und nicht umgesetzten Alkohol umfasst;
c. Abtrennen des homogenen Katalysatorsystems aus dem flüssigen Produktgemisch unter Erhalt eines Rohproduktgemisches, welches zumindest die durch die Alkoxycarbonylierung gebildeten Ester, die nicht umgesetzten Olefine und nicht umgesetzte Alkohole umfasst; und
d. Destillative Aufarbeitung des Rohproduktgemisches in mindestens einer Destillationskolonne zur Abtrennung der nicht umgesetzten Alkohole und der nicht umgesetzten Olefine unter Erhalt eines Estergemisches, welches die gebildeten Ester enthält.

2. Verfahren nach Anspruch 1, wobei die Alkoxycarbonylierung in Schritt b bei einer Temperatur von 60 bis 120 °C, vorzugsweise 65 bis 110 °C, besonders bevorzugt 70 bis 100 °C durchgeführt wird.

3. Verfahren nach Anspruch 1 oder 2, wobei die Alkoxycarbonylierung in Schritt b bei dem Kohlenmonoxiddruck von 10 bis 35 bar, vorzugsweise 12,5 bis 30 bar, besonders bevorzugt 15 bis 25 bar durchgeführt wird.

4. Verfahren nach einem der vorhergehenden Ansprüche, wobei der Gehalt des Metalls der Gruppe 8 bis 10 des Periodensystems der Elemente, insbesondere des Palladiums, in der der Reaktionslösung bei der Alkoxycarbonylierung in Schritt b 100 bis 500 ppm, vorzugsweise 150 bis 450 ppm, besonders bevorzugt 180 bis 350 ppm beträgt.

5. Verfahren nach einem der vorhergehenden Ansprüche, wobei der Alkohol ein einwertiger Alkohol mit 1 bis 4 Kohlenstoffatomen, insbesondere Methanol, Ethanol, Propanol oder Butanol ist.

6. Verfahren nach einem der vorhergehenden Ansprüche, wobei der Anteil von 2,4,4-Trimethylpent-1-en im Di-isobutenstrom mindestens 60 Mol%, vorzugsweise mindestes 70 Mol% beträgt.

7. Verfahren nach einem der vorhergehenden Ansprüche, wobei die Abtrennung des homogenen Katalysatorsystems in Schritt c mittels Membrantrennung erfolgt.

8. Verfahren nach Anspruch 7, wobei das homogene Katalysatorsystem im Retentat angereichert wird.

9. Verfahren nach einem der vorhergehenden Ansprüche, wobei der Di-isobutenstrom, der Olefinstrom, der Alkohol und das homogene Katalysatorsystem, zunächst in einem Mischbehälter vermischt werden, bevor sie in die Reaktionszone geleitet werden.

10. Verfahren nach einem der vorhergehenden Ansprüche, wobei die destillative Aufarbeitung in Schritt d in einer einzigen Destillationskolonne erfolgt.

11. Verfahren nach Anspruch 10, wobei der Druck in der Destillationskolonne im Bereich von 0,3 bis 2 bar, bevorzugt im Bereich von 0,4 bis 1 bar besonders bevorzugt im Bereich von 0,5 bis 0,7 bar liegt.

12. Verfahren nach Anspruch 10 oder 11, wobei die Temperatur im Sumpf der Destillationskolonne in einem Bereich von 80 °C bis 160 °C liegt.

13. Verfahren nach einem der Ansprüche 10 bis 12, wobei die Temperatur am Kopf der Destillationskolonne in einem Bereich von 30 bis 80 °C liegt.

14. Verfahren nach einem der vorhergehenden Ansprüche, wobei ein C4-Olefin und Methanol eingesetzt werden, wodurch als Estergemisch eine Mischung aus 3,5,5-Trimethylhexansäuremethylester und Valeriansäuremethylester, 2-Methylbuttersäuremethylester, 3-Methylbuttersäuremethylester oder einer Mischung daraus erhalten wird.

15. Verfahren nach einem der vorhergehenden Ansprüche, wobei in Schritt d nicht umgesetzter Alkohol und nicht umgesetzte Olefine abgetrennt und zur Alkoxycarbonylierung in Schritt b zurückgeführt werden.

## Claims

1. Process for alkoxycarbonylation of diisobutene and a C4 to C7 olefin, wherein the process comprises at least the following steps:
a. providing a diisobutene stream containing 2,4,4-trimethylpent-2-ene and 2,4,4-trimethylpent-1-ene and providing an olefin stream containing the C4 to C7 olefin;
b. alkoxycarbonylation of diisobutene and the C4 to C7 olefin with an alcohol and carbon monoxide in the presence of a homogeneous catalyst system comprising at least one metal of group 8 to 10 of the periodic table of the elements or a compound thereof, a phosphorus-containing ligand and an acid in a common reaction zone to obtain a liquid product mixture comprising at least the esters formed by the alkoxycarbonylation, the homogeneous catalyst system, unreacted olefins and unreacted alcohol;
c. removing the homogeneous catalyst system from the liquid product mixture to obtain a crude product mixture comprising at least the esters formed by the alkoxycarbonylation, the unreacted olefins and unreacted alcohols; and
d. distillative processing of the crude product mixture in at least one distillation column to remove the unreacted alcohols and the unreacted olefins to obtain an ester mixture containing the esters formed.

2. Process according to Claim 1, wherein the alkoxycarbonylation in step b is performed at a temperature of 60°C to 120°C, preferably 65°C to 110°C, particularly preferably 70°C to 100°C.

3. Process according to Claim 1 or 2, wherein the alkoxycarbonylation in step b is performed at a carbon monoxide pressure of 10 to 35 bar, preferably 12.5 to 30 bar, particularly preferably 15 to 25 bar.

4. Process according to any of the preceding claims, wherein the content of the metal of group 8 to 10 of the periodic table of the elements, in particular of palladium, in the alkoxycarbonylation reaction solution in step b is 100 to 500 ppm, preferably 150 to 450 ppm, particularly preferably 180 to 350 ppm.

5. Process according to any of the preceding claims, wherein the alcohol is a monohydric alcohol having 1 to 4 carbon atoms, especially methanol, ethanol, propanol or butanol.

6. Process according to any of the preceding claims, wherein the proportion of 2,4,4-trimethylpent-1-ene in the diisobutene stream is at least 60 mol%, preferably at least 70 mol%.

7. Process according to any of the preceding claims, wherein the removal of the homogeneous catalyst system in step c is effected by membrane separation.

8. Process according to Claim 7, wherein the homogeneous catalyst system accumulates in the retentate.

9. Process according to any of the preceding claims, wherein the diisobutene stream, the olefin stream, the alcohol and the homogeneous catalyst system are initially mixed in a mixing vessel before they are passed into the reaction zone.

10. Process according to any of the preceding claims, wherein the distillative processing in step d is carried out in a single distillation column.

11. Process according to Claim 10, wherein the pressure in the distillation column is in the range from 0.3 to 2 bar, preferably in the range from 0.4 to 1 bar, particularly preferably in the range from 0.5 to 0.7 bar.

12. Process according to Claim 10 or 11, wherein the temperature in the bottom of the distillation column is in a range from 80°C to 160°C.

13. Process according to any of Claims 10 to 12, wherein the temperature at the top of the distillation column is in a range from 30°C to 80°C.

14. Process according to any of the preceding claims, wherein a C4 olefin and methanol are employed, as a result of which the ester mixture obtained is a mixture of methyl 3,5,5-trimethylhexanoate and methyl valerate, methyl 2-methylbutyrate, methyl 3-methylbutyrate or a mixture thereof.

15. Process according to any of the preceding claims, wherein in step d unreacted alcohol and unreacted olefins are separated and recycled to the alkoxycarbonylation in step b.

## Revendications

1. Procédé d'alcoxycarbonylation de diisobutène et d'une C4-C7-oléfine, le procédé comprenant au moins les étapes suivantes :
a. fourniture d'un flux de diisobutène contenant du 2,4,4-triméthylpent-2-ène et du 2,4,4-triméthylpent-1-ène et fourniture d'un flux d'oléfine contenant la C4-C7-oléfine ;
b. alcoxycarbonylation de diisobutène et de la C4-C7-oléfine avec un alcool et du monoxyde de carbone en présence d'un système catalytique homogène, qui comprend au moins un métal du 8e au 10e groupe du système périodique des éléments ou un composé de celui-ci, un ligand contenant du phosphore et un acide, dans une zone de réaction commune avec obtention d'un mélange produit liquide, qui comprend au moins l'ester formé par l'alcoxycarbonylation, le système catalytique homogène, des oléfines non transformées et de l'alcool non transformé ;
c. séparation du système catalytique homogène à partir du mélange produit liquide avec obtention d'un mélange produit brut, qui comprend au moins l'ester formé par l'alcoxycarbonylation, les oléfines non transformées et les alcools non transformés ; et
d. traitement par distillation du mélange produit brut dans au moins une colonne de distillation pour la séparation des alcools non transformés et des oléfines non transformées avec obtention d'un mélange d'esters, qui contient l'ester formé.

2. Procédé selon la revendication 1, dans lequel l'alcoxycarbonylation dans l'étape b est réalisée à une température de 60 à 120°C, de préférence de 65 à 110°C, de manière particulièrement préférée de 70 à 100°C.

3. Procédé selon la revendication 1 ou 2, dans lequel l'alcoxycarbonylation dans l'étape b est réalisée à une pression de monoxyde de carbone 10 à 35 bars, de préférence de 12,5 à 30 bars, de manière particulièrement préférée de 15 à 25 bars.

4. Procédé selon l'une des revendications précédentes, dans lequel la teneur en métal du 8e au 10e groupe du système périodique des éléments, en particulier en palladium, dans la solution réactionnelle lors de l'alcoxycarbonylation dans l'étape b est de 100 à 500 ppm, de préférence de 150 à 450 ppm, de manière particulièrement préférée de 180 à 350 ppm.

5. Procédé selon l'une des revendications précédentes, dans lequel l'alcool est un alcool monovalent comprenant 1 à 4 atomes de carbone, en particulier le méthanol, l'éthanol, le propanol ou le butanol.

6. Procédé selon l'une des revendications précédentes, dans lequel la proportion de 2,4,4-triméthylpent-1-ène dans le flux de diisobutène est d'au moins 60% en mole, de préférence d'au moins 70% en mole.

7. Procédé selon l'une des revendications précédentes, dans lequel la séparation du système catalytique homogène dans l'étape c s'effectue au moyen d'une séparation par membrane.

8. Procédé selon la revendication 7, dans lequel le système catalytique homogène s'accumule dans le rétentat.

9. Procédé selon l'une des revendications précédentes, dans lequel le flux de diisobutène, le flux d'oléfines, l'alcool et le système catalytique homogène sont d'abord mélangés dans un récipient de mélange avant d'être guidés dans la zone de réaction.

10. Procédé selon l'une des revendications précédentes, dans lequel le traitement par distillation dans l'étape d s'effectue dans une seule colonne de distillation.

11. Procédé selon la revendication 10, dans lequel la pression dans la colonne de distillation se situe dans la plage de 0,3 à 2 bars, de préférence dans la plage de 0,4 à 1 bar, de manière particulièrement préférée dans la plage de 0,5 à 0,7 bar.

12. Procédé selon la revendication 10 ou 11, dans lequel la température dans le fond de la colonne de distillation se situe dans une plage de 80°C à 160°C.

13. Procédé selon l'une des revendications 10 à 12, dans lequel la température en tête de la colonne de distillation se situe dans une plage de 30 à 80°C.

14. Procédé selon l'une des revendications précédentes, dans lequel une C4-oléfine et du méthanol sont utilisés, suite à quoi on obtient, comme mélange d'esters, un mélange de l'ester méthylique de l'acide 3,5,5-triméthylhexanoïque et l'ester méthylique de l'acide valérianique, l'ester méthylique de l'acide 2-méthylbutyrique, l'ester méthylique de l'acide 3-méthylbutyrique ou un mélange de ceux-ci.

15. Procédé selon l'une des revendications précédentes, dans lequel, dans l'étape d, l'alcool non transformé et les oléfines non transformées sont séparés et renvoyées à l'alcoxycarbonylation dans l'étape b.
